# EUROPÄISCHE PATENTSCHRIFT

(11) **EP 0 688 546 B1**
(45) Veröffentlichungstag und Bekanntmachung des Hinweises auf die Patenterteilung: **12.12.2001**
(21) Anmeldenummer: 94810353.6
(22) Anmeldetag: 15.06.1994
(51) Int. Cl.: A61F 2/34

(54) **Künstliche Hüftgelenkpfanne**
Artificial acetabular cup
Coque acétabulaire artificielle

(43) Veröffentlichungstag der Anmeldung: 27.12.1995
(73) Patentinhaber: Sulzer Orthopädie AG, 6340 Baar (CH)
(72) Erfinder: Wagner, Heinz, Prof. Dr. med., Orthopädische, D-90592 Schwarzenbruck (DE); Willi, Roland, CH-8413 Neftenbach (CH); Stutz, Heinrich, CH-8500 Frauenfeld (CH)
(74) Vertreter: Sulzer Management AG

(56) Entgegenhaltungen:
- EP-A- 0 237 751
- EP-A- 0 239 485
- EP-A- 0 242 633
- EP-A- 0 265 712
- EP-A- 0 563 503
- WO-A-92/22265

## Beschreibung

Die Erfindung betrifft eine künstliche Hüftgelenkpfanne gemäss dem Oberbegriff von Anspruch 1.

Die Erfindung betrifft weiter ein Verfahren zum Zusammenstellen sowie zum Ausrichten der erfindungsgemässen Hüftgelenkpfanne.

Aus der EP-A-0 563 503 ist eine Stützvorrichtung für eine künstliche Hüftgelenkpfanne bekannt, die eine halbkugelförmige Stützschale umfasst, welche an ihrem äquatorialen Rand Stützlappen mit Durchbrechungen aufweist. Eine solche Stützvorrichtung wird vorzugsweise implantiert, wenn der Beckenknochen an der das Hüftgelenk zu tragenden Stelle beschädigt oder stark degeneriert ist, was insbesondere bei Reoperationen häufig der Fall ist. Die Stützlappen werden während dem Implantieren durch plastische Deformation dem Verlauf des Beckenknochens angepasst, sodass die Stützvorrichtung mit durch die Stützlappen verlaufenden Knochenschrauben in tragfähigen Bereichen des Beckenknochens verankerbar ist. Daraufhin wird ein Knochenzement appliziert, der den Zwischenraum zwischen der Stützschale und dem Knochengewebe ausfüllt und innerhalb der Stützschale ein Zementbett bildet. Weiter wird eine Innenschale in die Stützschale eingesetzt, wobei die Innenschale in einer orthopädisch günstigen Lage positioniert wird, und die gegenseitige Lage durch den aushärtenden Knochenzement fixiert wird.

Ein Nachteil einer solchen Hüftgelenkpfanne ist darin zu sehen, dass zur Befestigung und Ausrichtung der Innenschale ein Knochenzement erforderlich ist. Die Verwendung von Knochenzement weist bekanntlich mehrere Nachteile auf, unter anderem den, dass der Beckenknochen bei einer Reoperation in ungünstigen Fällen eine übermässige Schädigung erfährt.

Aus der WO-A-92/22265 ist eine künstliche Hüftgelenkpfanne bekannt, welche eine Aussenschale und eine in diese Aussenschale einsetzbare Innenschale aufweist. Die Innenschale weist einen Einsatz mit einer Artikulationsfläche auf. Die Aussenschale ist mit Schlitzen versehen, die etwas geneigt zur Umfangrichtung verlaufen. In diese Schlitze greifen beim Einsetzen der Innenschale Zapfen ein, die von der Aussenfläche der Innenschale abstehen. Die Innenschale mitsamt dem Einsatz wird nach Art eines Bajonettverschlusses in die Aussenschale eingesetzt.

Aus der EP-A-0,242,633 ist eine künstliche Hüftgelenkpfanne bekannt, welche eine Aussenschale und eine in diese Aussenschale einsetzbare Innenschale aufweist. Die Aussenschale ist mit Meridianschlitzen versehen, welche vom Äquator weg in Richtung zum Pol hin verlaufen und die Aussenschale in mehrere Lappen unterteilt. Auf ihrer Innenwand ist die Aussenschale mit einem Gewinde versehen. Die Innenschale ist auf ihrer Aussenwand mit einem entsprechenden Gegengewinde versehen. Zum Einsetzen der Innenschale in die Aussenschale wird die Innenschale in die Aussenschale eingeschraubt, wobei die Lappen der Aussenschale gespreizt werden.

Der Erfindung liegt die Aufgabe zugrunde, eine Hüftgelenkpfanne zu schaffen, die ohne Knochenzement implantierbar ist, wobei die gegenseitige Lage von Stützschale und Innenschale während dem Implantieren bestimmbar ist.

Diese Aufgabe wird erfindungsgemäss gelöst durch den Gegenstand mit den in Anspruch 1 definierten Merkmalen. Die Unteransprüche 2 bis 7 beziehen sich auf weitere vorteilhafte Ausführungsformen. Die Erfindung wird weiter gelöst mit einem Verfahren zum Zusammenstellen einer erfindungsgemässen Hüftgelenkpfanne gemäss Anspruch 8. Der Unteranspruch 9 bezieht sich auf einen weiteren, vorteilhaften Verfahrensschritt.

Die erfindungsgemässe Hüftgelenkpfanne umfasst eine Stützschale sowie eine darin einführbare Innenschale. Die Innenschale weist eine halbkugelförmige Aussenfläche auf und die Stützschale eine halbkugelförmige Innenfläche. Die Innenfläche der Stützschale weist Haltemittel auf und die Aussenfläche der Innenschale weist mindestens eine wendelförmig verlaufende Ausnehmung auf. Die Innenschale ist mit einer schraubenartigen Bewegung in die Stützschale einführbar, um die Haltemittel und die Ausnehmung in eine Wirkverbindung zu bringen und die gegenseitige Lage von Stützschale und Innenschale festzulegen. Die Haltemittel sind durch mindestens drei von der Innenfläche der Stützschale vorstehende Nocken gebildet, wobei alle Nocken in einer Ebene liegend angeordnet sind.

Vorteile der Erfindung sind darin zu sehen, dass die Innenschale und die Stützschale unter verschiedenen, wählbaren Neigungswinkeln verbindbar sind, dass die Verbindung keinen Knochenzement erfordert, und dass die Verbindung mit einer einfachen, schraubenförmigen Drehbewegung auch während der Implantation möglich ist, sodass der Neigungswinkel auch während der Implantation noch einstellbar beziehungsweise korrigierbar ist.

Im folgenden wird die Erfindung anhand von Ausführungsbeispielen beschrieben. Es zeigen:
- Fig. 1: Einen Längsschnitt durch eine künstliche Hüftgelenkpfanne;
- Fig. 2: einen Längsschnitt durch eine äussere Stützschale bei eingesetzter Innenschale;
- Fig. 3: eine Untenansicht einer äusseren Stützschale;
- Fig. 4: einen Längsschnitt durch eine äussere Stützschale;
- Fig. 5: ein Ausführungsbeispiel eines Haltemittels;
- Fig. 6a-d: Ausführungsbeispiele von schwenkbaren Haltemitteln;
- Fig. 7a-b: Ausführungsbeispiele von Verdrehsicherungen.

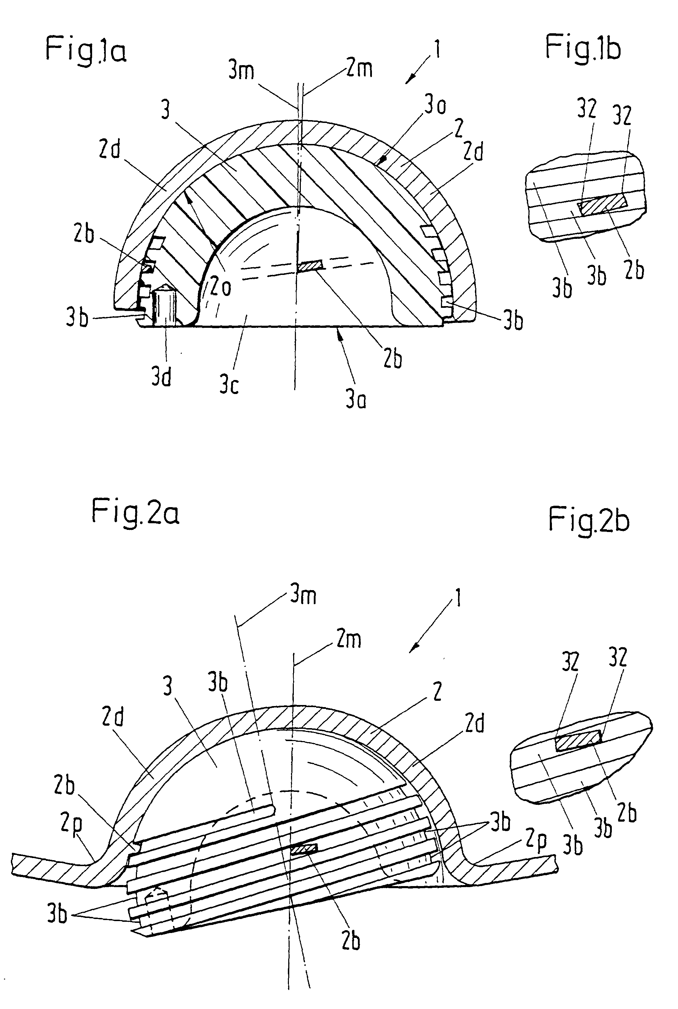

Fig. 1a zeigt eine Hüftgelenkpfanne 1, die eine äussere Stützvorrichtung 2 umfasst, welche als eine halbkugelförmige Stützschale 2d mit einer Achse 2m ausgebildet ist, sowie eine halbkugelförmige Innenschale 3 mit einer Achse 3m. Die Innenfläche 2o der Stützschale 2d weist drei in den Innenraum der Stützschale 2d vorstehende Haltemittel 2b auf, wobei aus der Figur 1a die Anordnung zweier Haltemittel 2b sichtbar sind. Die Aussenfläche 3o der Innenschale 3 weist eine wendelförmig verlaufende Ausnehmung 3b auf. Beim Zusammenfügen der Aussenschale 2b mit der Innenschale 3 ist die gegenseitige Lage der Achsen 2m, 3m in einem weiten Bereich wählbar, indem die Innenschale 3 vorerst in einer bezüglich der Stützschale 2b gewünschten Lage positioniert wird und daraufhin die Innenschale 3 durch eine um die Achse 2m erfolgenden Drehbewegung in die Stützschale 2b hineingedreht wird, wobei die wendelförmige Ausnehmung 3b in die Haltemittel 2b greift, sodass sich eine gegenseitige Wirkverbindung ergibt. Im dargestellten Ausführungsbeispiel ist die Innenschale 3 vollständig in die Stützschale 2b hineingedreht, sodass die Innenfläche 2o der Stützschale 2b unter Vorspannung auf der Aussenfläche 3o der Innenschale 3 aufliegt, was eine in Richtung der Achse 3m verlaufende Reaktionskraft erzeugt, die von den Haltemitteln 2b aufgenommen wird, sodass die Innenschale 3 fest in der Stützschale 2d verankert ist. In vorliegenden Ausführungsbeispiel weist das Haltemittel 2b, wie in Fig. 1b mit einer Aufsicht auf die Aussenfläche 3o dargestellt, einen rechteckigen Querschnitt auf, der gegenüber dem Verlauf der Ausnehmung 3b leicht versetzt verläuft, sodass sich zwischen der Innenschale 3 und der Stützschale 2d sehr kleine Berührungsflächen 32 ergeben. Die dadurch entstehende hohe Flächenbelastung kann genutzt werden, um eine Sicherung gegen ein Verdrehen der beiden Schalen 3, 2d zu bewirken, indem zum Beispiel das Haltemittel 2b aus einem Metall und die Innenschale 3 aus einem Kunststoff wie Polyäthylen ausgeführt ist, sodass sich das Haltemittel 2b an der Berührungsfläche 32 in den Kunststoff eingräbt und die Innenschale 3 plastisch deformiert, was ein gegenseitiges Verdrehen der Schalen 3, 2d behindert oder verhindert.

Die Innenschale 3 weist eine kreisförmige Öffnung 3a auf sowie einen kugelförmigen Innenraum 3c zur Aufnahme eines Gelenkkugelkopfes. Weiter weist die Innenschale 3 eine Ausnehmung 3d auf zur Aufnahme eines Setzwerkzeuges, um die Innenschale 3 mit einer Drehbewegung in die Stützschale 2d einzuschrauben. Die dargestellte Stützschale 2d ist mittels Knochenschrauben am Beckenknochen fixierbar, wobei die entsprechenden Ausnehmungen in der Stützschale 2d nicht dargestellt sind.

Die Figuren 2, 3 und 4 zeigen ein weiteres Ausführungsbeispiel einer metallischen Stützvorrichtung 2, die eine halbkugelförmige Stützschale 2d sowie an ihrem äquatorialen Rand abstehende Stützlappen 2a aufweist. Wie in der Beschreibungseinleitung erwähnt, wird eine solche Stützvorrichtung 2 mit durch Ausnehmungen 2q der Stützlappen 2a verlaufenden Knochenschrauben am Beckenknochen befestigt. Dabei wird auf den Zustand des Beckenknochens Rücksicht genommen, sodass es möglich ist, die Ausrichtung der Stützschale 2d in ein orthopädisch ungünstige Stellung zu liegen kommt. Dies lässt sich korrigieren, indem nun die Innenschale 3 in einer orthopädisch günstigen Lage in der Stützschale 2d befestigt wird. Ein solches Vorgehen während dem Implantieren weist den Vorteil auf, dass ein Operateur in einem ersten Schritt die Stützvorrichtung 2 optimal am Beckenknochen befestigen kann, weil bezüglich der Ausrichtung der Stützvorrichtung 2 eine grosse Toleranz besteht, und dass in einem zweiten Schritt die Innenschale 3 orthopädisch günstig ausgerichtet wird. In Fig. 2a ist eine Hüftgelenkpfanne 1 dargestellt, deren Achse 3m der Innenschale 3 eine grössere Abweichung von der Achse 2m der Stützschale 2d aufweist. Die Innenschale 3 ist in einer Seitenansicht dargestellt, wobei die Aussenfläche 3o eine einzige, spiralförmig verlaufende Ausnehmung 3b aufweist. Auf Fig. 2b ist das Zusammenwirken des Haltemittels 2b mit der Ausnehmung 3b dargestellt, wobei sich durch die Form und Anordnung des Haltemittels 2b wiederum kleine Berührungsflächen 32 ergeben.

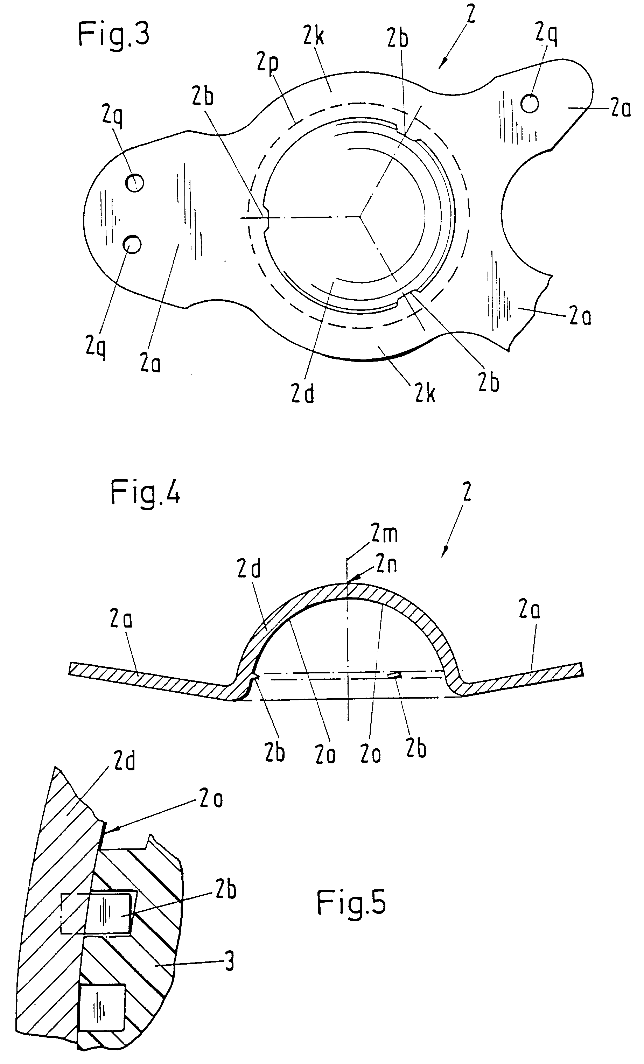

Die Unteransicht der metallischen Stützvorrichtung 2 gemäss Fig. 3 zeigt die Anordnung der drei Haltemittel 2b, die im Bereich des äquatorialen Randes 2p gleichmässig über den Umfang verteilt sind und in den Innenraum der Stützschale 2d vorstehen. Aus der Seitenansicht gemäss Fig. 4 ist ersichtlich, dass die Haltemittel 2b in einer Ebene liegend angeordnet sind, wobei die Ausrichtung der einzelnen Haltemittel 2b einen Neigungswinkel bezüglich dieser Ebene aufweisen. Die Haltemittel 2b haben den Zweck in eine Ausnehmung 3b der Innenschale 3 einzugreifen, um diese zu halten, wobei die Innenschale 3 in einer Vielzahl von Stellungen in die Stützschale 2d einführbar sein sollte. Um diesen Zweck zu erfüllen gibt es eine Vielzahl möglicher Anordnungen der Haltemittel 2b innerhalb der Innenfläche 2o die günstig sind. Ebenso kann es sich als vorteilhaft erweisen, mehr als drei Haltemittel 2b an der Innenfläche 2o anzuordnen. Auch für die Ausgestaltung der Form des Haltemittels 2b gibt es eine Vielzahl von Möglichkeiten, um den erforderlichen Zweck zu erfüllen. Fig. 5 zeigt einen Querschnitt durch eine Innenschale 3 sowie eine Stützschale 2b mit einem weiteren Ausführungsbeispiel eines Haltemittels 2b. Das Haltemittel 2b ist als zylinderförmiger Körper ausgebildet, der in eine entsprechend angepasse Ausnehmung an der Innenfläche 2o der Stützschale 2d eingelassen ist. Die Innenfläche 2o der Stützschale 2d kann eine Mehrzahl von Ausnehmungen für ein Haltemittel 2b aufweisen, sodass die Haltemittel 2b auch unmittelbar vor oder während der Implantation an der Stützschale 2d angeordnet werden können. Dadurch lässt sich die gegenseitige Lage von Stützschale 2d und Innenschale 3 noch differenzierter beeinflussen.

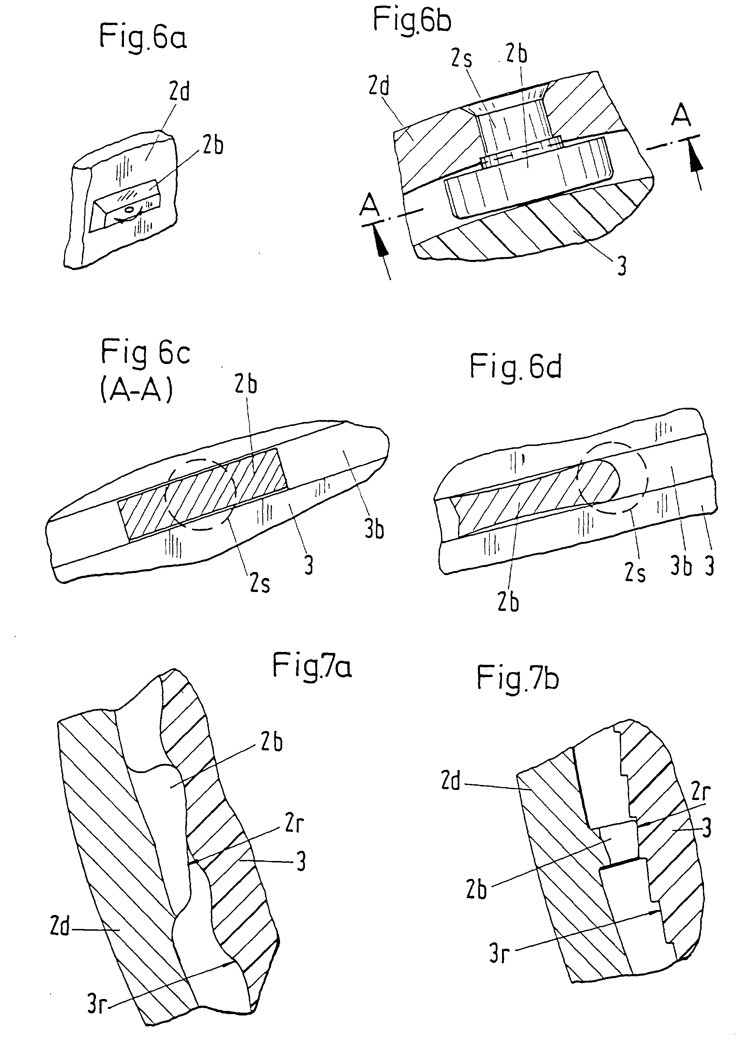

Die Figuren 6a bis 6d zeigen weitere Ausführungsformen von Haltemitteln 2b, die alle die Eigenschaft aufweisen, dass sie, wie in Fig. 6a mit einem Pfeil dargestellt, drehbar an der Stützschale 2d gelagert sind, sodass sich die Ausrichtung des Haltemittels 2b dem Verlauf der Ausnehmung 3b des Innenschale 3 anpasst. Fig. 6b zeigt einen Schnitt durch ein drehbar gelagertes Haltemittel 2b, das ein zylinderförmiges Teil 2s aufweist, das in einer Durchbrechung der Stützschale 2d liegt, und dadurch bezüglich der Stützschale 2d drehbar ist. Weiter ist die Lage der Innenschale 3 dargestellt. Ein Schnitt entlang der Linie A-A ist in Fig. 6c dargestellt, woraus ersichtlich ist, dass das Haltemittel 2b symmetrisch ausgestaltet ist und seine Lage dem Verlauf der Ausnehmung 3b anpasst. Fig. 6d zeigt aus einer gleichen Ansicht wie Fig. 6c ein weiteres Ausführungsbeispiel eines Haltemittels 2b, das mit dem zylinderförmigen Teil 2s, der das Drehzentrum definiert, asymmetrisch verbunden ist. Das dem Teil 2s gegenüberliegende Ende des Haltemittels 2b ist schwalbenschwanzförmig ausgebildet und weist zwei ausgeprägte Kanten auf. Diese Kanten können als eine Sicherung gegen ein Verdrehen der Innenschale 3 dienen. Bei einem Zurückdrehen der Innenschale 3 können sich eine oder beide Kanten des Haltemittels 2b in die Innenschale 3 eingraben, was eine Verdrehsicherung bewirkt. Eine asymmetrische Ausgestaltung des Haltemittels 2b fördert des Verhalten des Haltemittels, sich beim zurückdrehen in die Innenschale 3 einzugraben.

Die Figuren 7a und 7b zeigen einen Schnitt durch eine Stützschale 2d mit Haltemittel 2b sowie eine Innenschale 3. Der Verlauf der Oberfläche 2r des Haltemittels 2b sowie der Oberfläche 3r der Innenschale 3 sind derart aufeinander angepasst, dass ein Verdrehen der Stützschale 2d relativ zur Innenschale 3 möglichst verhindert wird, um eine Sicherung gegen Verdrehen auszubilden. In Fig. 7a weist die Oberfläche 3r eine Welligkeit auf, die bezüglich der Oberfläche 2r derart ausgestaltet ist, dass ein Verdrehen behindert wird, aber unter Anlegen eines grösseren Drehmomentes möglich ist. Fig. 7b zeigt eine Oberfläche 3r mit einer sägezahnförmigen Ausgestaltung, in die das entsprechend ausgebildete Haltemittel 2b eingreift. Mit dieser Ausführungsform lässt sich eine Innenschale 3 unlösbar mit einer Stützschale 2d verbinden. Unter Anlegung eines sehr grossen Drehmomentes könnte die Innenschale 3 eventuell wieder lösbar sein, die Oberfläche 3r der Innenschale 3 würde dabei jedoch partiell zerstört werden.

## Patentansprüche

1. Künstliche Hüftgelenkpfanne (1), umfassend eine Stützschale (2; 2d) sowie eine darin einführbare Innenschale (3), wobei die Innenschale (3) eine halbkugelförmige Aussenfläche (30) aufweist, und die Stützschale (2;2d) eine halbkugelförmige Innenfläche (2o), wobei die Innenfläche (20) der Stützschale (2;2d) Haltemittel und die Aussenfläche (30) der Innenschale mindestens eine wendelförmig verlaufende Ausnehmung (3b) aufweist, und dass die Innenschale (3) mit einer schraubenartigen Bewegung in die Stützschale (2;2d) einführbar ist, um die Haltemittel und die Ausnehmung (3b) in eine Wirkverbindung zu bringen und die gegenseitige Lage von Stützschale (2;2d) und Innenschale festzulegen, **dadurch gekennzeichnet, dass** die Haltemittel durch mindestens drei von der Innenfläche (20) der Stützschale (2) vorstehende Nocken (2b) ausgebildet sind, wobei alle Nocken (2b) in einer Ebene liegend angeordnet sind.

2. Künstliche Hüftgelenkpfanne (1) nach Anspruch 1, **dadurch gekennzeichnet, dass** alle Nocken (2b) in einer senkrecht zu einer Achse (2m) der Stützschale (2;2d) verlaufenden Ebene angeordnet sind.

3. Künstliche Hüftgelenkpfanne (1) nach einem der Ansprüche 1 oder 2, **dadurch gekennzeichnet, dass** die Nocken (2b) schwenkbar mit der Stützschale (2; 2d) verbunden sind, sodass sich die Lage der Nocken(2b) dem Verlauf der Ausnehmung (3b) anpasst.

4. Künstliche Hüftgelenkpfanne (1) nach Anspruch 3, **dadurch gekennzeichnet, dass** die jeweilige Nocke (2b) eine Drehzentrum aufweist und bezüglich dem Drehzentrum asymmetrisch ausgebildet ist.

5. Künstliche Hüftgelenkpfanne (1) nach einem der Ansprüche 1 bis 4, **dadurch gekennzeichnet, dass** die Innenfläche (2o) der Stützschale (2; 2d) Ausnehmungen zur Aufnahme der Nocken (2b) aufweist.

6. Künstliche Hüftgelenkpfanne (1) nach einem der Ansprüche 1 bis 5, **dadurch gekennzeichnet, dass** die Nocken (2b) und die Ausnehmung (3b) eine derartig ausgebildete Oberflächenstruktur aufweisen, dass eine gegenseitige schraubenartige Bewegung in mindestens einer Drehrichtung behindert wird, um die Innenschale (3) in der Stützschale (2;2d) zu arretieren.

7. Künstliche Hüftgelenkpfanne (1) nach einem der vorhergehenden Ansprüche, **dadurch gekennzeichnet, dass** die Stützvorrichtung (2) aus einem Metall und die Innenschale (3) aus einem Metall, einem Kunststoff oder einer Keramik gefertigt ist.

8. Verfahren zum Zusammenstellen einer Hüftgelenkpfanne (1) gemäss einem der Ansprüche 1 bis 7, **dadurch gekennzeichnet,**
- **dass** die Innenschale (3) an die Stützschale (2; 2d) angelegt und die gegenseitige Lage festgelegt wird,
- **dass** die Innenschale (3) mit einer schraubenförmigen Drehbewegung in die Stützschale (2; 2d) gedreht wird, und dabei die Ausnehmung (3b) mit den Nocken (2b) in Wirkverbindung gebracht wird,
- und **dass** die Innenschale (3) gedreht wird, bis die Innenschale (3) fest in der Stützschale (2;2d) arretiert ist.

9. Verfahren zum Zusammenstellen einer Hüftgelenkpfanne (1) gemäss Anspruch 8, **dadurch gekennzeichnet, dass** die Innenschale (3) gedreht wird, bis auf die Innenschale (3) eine in Richtung der Achse (3m) wirkenden Kraft erzeugt wird, sodass zwischen der Ausnehmung (3b) und den Nocken (2b) eine in Richtung der Achse (3m) wirkende Reaktionskraft erzeugt wird.

## Claims

1. Artificial acetabulum (1), comprising a support shell (2; 2d) and also an inner shell (3) introducible therein, wherein the inner shell (3) has a hemispherical outer surface (30) and the support shell (2; 2d) has a hemispherical inner surface (20), wherein the inner surface (20) of the support shell (2; 2d) has retaining means and the outer surface (30) of the inner shell has at least one helically extending recess (3b) and wherein the inner shell (3) can be introduced with a screw-like movement into the support shell (2; 2d) in order to bring the retaining means and the recess (3b) into an operative connection and to fix the mutual position of the support shell (2; 2d) and the inner shell ,
**characterised in that** the retaining means are formed by at least three lugs (2b) which project from the inner surface (20) of the support shell (2), with all lugs (2b) being arranged lying in one plane.

2. Artificial acetabulum (1) in accordance with claim 1, **characterised in that** all lugs (2b) are arranged in a plane which extends perpendicular to an axis (2m) of the support shell (2; 2d).

3. Artificial acetabulum (1) in accordance with one of the claims 1 or 2, **characterised in that** the lugs (2b) are pivotally connected to the support shell (2; 2d), so that the position of the lugs (2b) adapts itself to the course of the recess (3b).

4. Artificial acetabulum (1) in accordance with claim 3, **characterised in that** the respective lug (2b) has a centre of rotation and is formed in an asymmetrical manner relative to the centre of rotation.

5. Artificial acetabulum (1) in accordance with one of the claims 1 to 4, **characterised in that** the inner surface (20) of the support shell (2; 2d) has recesses for the accommodation of the lugs (2b).

6. Artificial acetabulum (1) in accordance with one of the claims 1 to 5, **characterised in that** the lugs (2b) and the recess (3b) have a surface structure designed in such a way that a mutual screw motion in at least one direction of rotation is hindered in order to lock the inner shell (3) in the support shell (2; 2d).

7. Artificial acetabulum (1) in accordance with any one of the preceding claims, **characterised in that** the support device (2) is made of a metal and the inner shell (3) is made of a metal, a plastic or a ceramic material.

8. Method for the assembly of an acetabulum (1) in accordance with one of the claims 1 to 7, **characterised in that**
- the inner shell (3) is put against the support shell (2; 2d) and the mutual position is fixed,
- **in that** the inner shell (3) is rotated into the support shell (2; 2d) by a rotational screw motion, and the recess (2b) is thereby brought into operative connection with the lugs (3b),
- and **in that** the inner shell (3) is rotated until the inner shell (3) is firmly locked in the support shell (2; 2d).

9. Method for the assembly of an acetabulum (1) in accordance with claim 8, **characterised in that** the inner shell (3) is rotated until a force acting on the inner shell (3) in the direction of the axis (3m) is generated, so that a reaction force acting in the direction of the axis (3m) is generated between the recess (3b) and the lugs (2b).

## Revendications

1. Coque acétabulaire artificielle (1), comprenant une coque d'appui (2 ; 2d) ainsi qu'une coque interne (3) pouvant être insérée dans celle-ci, où la coque interne (3) présente une face externe demi-sphérique (30) et la coque d'appui (2 ; 2d) une face interne demi-sphérique (20), où la face interne (20) de la coque d'appui (2 ; 2d) présente des moyens de retenue et la face externe (30) de la coque interne au moins un évidement (3b) s'étendant hélicoïdalement, et en ce que la coque interne (3) peut être insérée avec un mouvement hélicoïdal dans la coque d'appui (2 ; 2d) pour amener les moyens de retenue et l'évidement (3b) en une liaison active et pour déterminer la position relative de la coque d'appui (2 ; 2d) et de la coque interne, **caractérisée en ce que** les moyens de retenue sont réalisés par au moins trois cames (2b) faisant saillie de la face interne (20) de la coque d'appui (2), où toutes les cames (2b) sont disposées pour se situer dans un plan.

2. Coque acétabulaire artificielle (1) selon la revendication 1, **caractérisée en ce que** toutes les cames (2b) sont disposées dans un plan s'étendant perpendiculairement à un axe (2m) de la coque d'appui (2 ; 2d).

3. Coque acétabulaire artificielle (1) selon l'une des revendications 1 ou 2, **caractérisée en ce que** les cames (2b) sont reliées d'une manière pivotante à la coque d'appui (2 ; 2d) de telle sorte que la position des cames (2b) s'adapte à l'allure de l'évidement (3b).

4. Coque acétabulaire artificielle (1) selon la revendication 3, **caractérisée en ce que** la came respective (2b) présente un centre de rotation et est réalisée d'une manière asymétrique relativement au centre de rotation.

5. Coque acétabulaire artificielle (1) selon l'une des revendications 1 à 4, **caractérisée en ce que** la face interne (20) de la coque d'appui (2 ; 2d) présente des évidements pour la réception des cames (2b).

6. Coque acétabulaire artificielle (1) selon l'une des revendications 1 à 5, **caractérisée en ce que** les cames (2b) et l'évidement (3b) présentent une structure de surface réalisée de façon qu'un mouvement hélicoïdal mutuel est empêché dans au moins une direction de rotation pour arrêter la coque interne (3) dans la coque d'appui (2 ; 2d).

7. Coque acétabulaire artificielle (1) selon l'une des revendications précédentes, **caractérisée en ce que** le dispositif d'appui (2) est fabriqué en un métal et la coque interne (3) en un métal, une matière synthétique ou une céramique.

8. Procédé pour composer une coque acétabulaire (1) selon l'une des revendications 1 à 7, **caractérisé**
- **en ce que** la coque interne (3) est appliquée à la coque d'appui (2 ; 2d) et que la position mutuelle est déterminée,
- **en ce que** la coque interne (3) est tournée avec un mouvement de rotation hélicoïdal dans la coque d'appui (2 ; 2d) et, ce faisant, l'évidement (3b) est amené en liaison active avec les cames (2b),
- et **en ce que** la coque interne (3) est tournée jusqu'à ce que la coque interne (3) soit arrêtée solidement dans la coque d'appui (2 ; 2d).

9. Procédé de composition d'une coque acétabulaire (1) selon la revendication 8, **caractérisé en ce que** la coque interne (3) est tournée jusqu'à ce que soit produite sur la coque interne (3) une force agissant dans la direction de l'axe (3m) de façon à ce que soit produite entre l'évidement (3b) et les cames (2b) une force de réaction agissant dans la direction de l'axe (3m).
